# FASCICULE DE BREVET EUROPEEN

(11) **EP 3 307 216 B1**
(45) Date de publication et mention de la délivrance du brevet: **31.07.2019**
(21) Numéro de dépôt: 16731242.0
(22) Date de dépôt: 07.06.2016
(51) Int. Cl.: A61F 5/01, B29C 51/00

(54) **ORTHESES SEMI-RIGIDES THERMOFORMABLES**
THERMOFORMBARE HALBSTEIFE ORTHESEN
THERMOFORMABLE SEMI-RIGID ORTHOSES

(30) Priorité: 10.06.2015 FR 1555282
(43) Date de publication de la demande: 18.04.2018
(73) Titulaire: Millet Innovation, 26270 Loriol sur Drome (FR)
(72) Inventeur: FONTAINE, Thierry, 26740 Marsanne (FR)
(74) Mandataire: de Roquemaurel, Bruno
(86) Numéro de dépôt international: PCT/FR2016/051355
(87) Numéro de publication internationale: WO 2016/198778

(56) Documents cités:
- WO-A1-2010/007243
- WO-A1-2011/111019
- WO-A1-2014/110029
- US-A- 4 572 167
- US-A- 4 716 892

## Description

La présente invention concerne des orthèses prévues pour limiter les mouvements d'un membre ou des orthèses correctrices telles que celles utilisées par exemple pour le traitement de l'Hallux Valgus par réaxation de l'articulation du gros orteil par effet mécanique.

Dans la conception d'orthèses légères, on peut identifier deux catégories de situations pour lesquelles il est requis de disposer d'éléments rigides adaptés à la forme du membre à maintenir. Dans certaines situations, il est requis d'immobiliser un membre. C'est le cas du pouce lorsqu'il s'agit de soulager les effets de la rhizarthrose. C'est aussi le cas lorsqu'un membre doit être maintenu dans une certaine position notamment en vue d'un traitement nécessitant une grande précision, par exemple pour positionner une personne sous un appareil de radiothérapie pour le traitement d'un cancer.

Pour traiter certains traumatismes articulaires notamment à la cheville ou au poignet, il peut être aussi requis d'assurer un certain maintien de l'articulation. Dans d'autres situations, il peut être également requis de mettre en tension une articulation dans un but correctif. C'est le cas par exemple du gros l'orteil pour traiter un hallux valgus. Dans le cas de certaines articulations comme la cheville, il peut être utile de prévenir des traumatismes par un maintien de l'articulation durant la pratique de certains exercices comme par exemple la marche. Un maintien peut compenser la laxité d'une articulation.

Pour répondre à des exigences de rigidité et d'adaptation à la forme du membre et/ou de l'articulation à maintenir, les orthésistes disposent de matériaux thermoformables à chaud, dont la forme peut être modifiée à volonté par de nouveaux formages à chaud. Une plaque en un tel matériau est généralement chauffée en l'immergeant pendant quelques minutes dans de l'eau chaude à une température déterminée supportable par le patient. La plaque devient alors suffisamment molle pour épouser la forme de la zone à maintenir, avant de durcir.

Actuellement, les orthésistes réalisent des orthèses sur mesure comprenant un matériau thermoformable, en respectant des procédures définies par les fabricants de ces matériaux. Il s'avère que toutes ces procédures ne peuvent pas être exécutées par le patient lui-même, et sont généralement prévues pour être mises en oeuvre par un professionnel. Par ailleurs, les orthèses réalisées en respectant ces procédures sont relativement lourdes et encombrantes, en raison notamment de l'usage de tricots lourds et épais et de bandes de fixation à boucles et à crochets (type Velcro).

D'après les documents WO 2014/110026, US 4 716 892 et US 4 572 167, il est connu d'utiliser ce type de matériau en le plaçant dans une poche généralement ouverte, associée à un système enveloppant ouvert que le praticien ferme en l'appliquant fortement sur le membre pour en obtenir la forme.

Il est donc souhaitable de réaliser une orthèse apte à assurer un maintien rigide d'un membre ou d'une articulation. Il peut être également souhaitable que cette orthèse soit plus légère et moins encombrante que les orthèses actuellement utilisées. Il peut être également souhaitable que cette orthèse puisse être adaptée et/ou utilisée sans nécessiter la participation d'un professionnel.

Des modes de réalisation concernent une orthèse pour le maintien d'un membre ou d'une articulation d'un humain ou d'un animal vertébré, comprenant : un manchon formé en un tissu élastique et conformé pour serrer un membre ou une articulation, une plaquette en un matériau thermoformable, et une poche formée sur le manchon pour recevoir la plaquette, la poche présentant une forme ajustée à celle de la plaquette. La surface de la plaquette et/ou la surface intérieure de la poche sont traitées pour amoindrir un effet d'encastrement de fils du tissu formant la poche, dans la plaquette, et ainsi autoriser un mouvement relatif de la plaquette par rapport au tissu formant la poche, pendant et après une opération de thermoformage de la plaquette, réalisée en plaçant l'orthèse sur le membre ou l'articulation avec la plaquette présente dans la poche.

Selon un mode de réalisation, la poche est formée par une couche fixée sur une face intérieure du manchon, destinée à venir en contact avec la peau du membre ou l'articulation à maintenir.

Selon un mode de réalisation, la poche est formée par une couche fixée sur le manchon, réalisée en un tissu rembourré.

Selon un mode de réalisation, la partie du manchon non recouverte par la poche est réalisée dans un tissu élastique.

Selon un mode de réalisation, le manchon est réalisé dans un tissu élastique présentant une épaisseur comprise entre 0,2 et 1,5 mm.

Selon un mode de réalisation, la plaquette comprend un film ou une enduction recouvrant le matériau thermoformable.

Selon un mode de réalisation, les faces intérieures de la poche destinées à venir en contact avec la plaquette ont subi un traitement de surface pour réduire leur adhérence avec la plaquette à la suite d'une opération de thermoformage de la plaquette.

Selon un mode de réalisation, le manchon est adapté à l'une des formes suivantes : la forme de la cheville et du pied d'un humain, avec une ouverture proximale pour le passage de la jambe, une ouverture distale pour le passage de l'avant du pied et une ouverture intermédiaire pour le passage du talon, le manchon comprenant deux poches destinées à couvrir les malléoles de la cheville en s'étendant entre la base du pied et l'ouverture proximale du manchon, les deux poches étant prévues pour recevoir deux plaquettes en un matériau thermoformable ; la forme du poignet d'un humain et d'une partie proximale de la main et du pouce, avec une ouverture distale pour le passage de la partie distale du pouce, une ouverture proximale pour le passage du bras et une ouverture intermédiaire pour le passage des doigts de la main, le manchon comprenant une poche destinée à couvrir l'ensemble du manchon à l'exception d'une partie du manchon destinée à couvrir le tranchant de la main, la poche étant prévue pour recevoir la plaquette en un matériau thermoformable ; la forme du pied et d'une partie proximale du gros orteil d'un humain, avec une ouverture distale pour le passage de la partie distale du gros orteil, une ouverture proximale pour le passage du pied et une ouverture intermédiaire pour le passage des autres orteils du pied, le manchon comprenant une poche sur une partie du manchon destinée à couvrir une partie de la face latérale interne du pied et gros orteil, la poche étant prévue pour recevoir la plaquette en un matériau thermoformable.

Selon un mode de réalisation, le manchon est adapté à la forme de la cheville et du pied d'un humain, le manchon étant solidaire d'une bande prévue pour être enroulée autour du pied et de la cheville, pour serrer davantage le manchon autour du pied et de la cheville, notamment durant le thermoformage de la plaquette.

Selon un mode de réalisation, une face externe de la poche, destinée à venir en contact avec la peau du membre ou l'articulation à maintenir, est recouverte d'une enduction en gel polymère, la partie de la poche enduite de gel polymère présentant une élasticité dans le sens perpendiculaire à l'axe du membre, supérieure ou égale celle dans un sens parallèle à l'axe du membre.

Selon un mode de réalisation, l'orthèse comprend une pastille en gel polymère fixée de manière amovible contre la face externe de la poche recouverte d'une enduction en gel polymère.

Des modes de réalisation peuvent également concerner un procédé de fabrication d'une orthèse pour le maintien d'un membre ou d'une articulation d'un humain ou d'un animal vertébré, comprenant des étapes consistant à : fabriquer un manchon en tissu élastique, ajusté pour serrer un membre ou une articulation, ajuster la forme d'une plaquette en un matériau thermoformable à celle d'une partie du membre ou de l'articulation, et former une poche sur le manchon pour recevoir la plaquette, la poche présentant une forme ajustée à celle de la plaquette. Le procédé comprend une étape de traitement de la surface de la plaquette et/ou de la surface intérieure de la poche pour amoindrir un effet d'encastrement de fils du tissu formant la poche, dans la plaquette, et ainsi autoriser un mouvement relatif de la plaquette par rapport au tissu formant la poche, pendant et après une opération de thermoformage de la plaquette, réalisée en plaçant l'orthèse sur le membre ou l'articulation avec la plaquette présente dans la poche.

Selon un mode de réalisation, la formation de la poche est réalisée en fixant une couche sur une face intérieure du manchon, destinée à venir en contact avec la peau du membre ou l'articulation à maintenir.

Des modes de réalisation peuvent également concerner un procédé de fabrication d'une orthèse pour le maintien d'un membre ou d'une articulation d'un humain ou d'un animal vertébré, comprenant des étapes de mise en oeuvre le procédé de fabrication défini précédemment, et de disposition de la plaquette dans la poche, ainsi qu'une opération de thermoformage de l'orthèse comprenant des étapes consistant à : chauffer l'orthèse à une température suffisante pour ramollir la plaquette, et placer le manchon autour du membre ou de l'articulation avant que la plaquette durcisse.

Selon un mode de réalisation, l'opération de thermoformage comprend une étape de couverture du membre ou de l'articulation d'un film avant de placer le manchon autour du membre ou de l'articulation.

Selon un mode de réalisation, l'opération de thermoformage comprend une étape de placement d'une pastille sur une zone de la face extérieure de la poche destinée à venir en contact avec la peau, avant de placer le manchon autour du membre ou de l'articulation, la pastille étant retirée du manchon après l'opération de thermoformage, de manière à ménager un espace vide entre la plaquette et la peau.

Des exemples de réalisation de l'invention seront décrits dans ce qui suit, à titre non limitatif en relation avec les figures jointes parmi lesquelles :
la figure 1 représente schématiquement en coupe une orthèse selon un mode de réalisation,
les figures 2A à 2F représentent schématiquement en coupe une partie de l'orthèse à différents instants d'une opération de thermoformage,
la figure 3 représente schématiquement la face latérale externe d'un pied et d'une cheville, équipés d'une orthèse de cheville selon un mode de réalisation,
la figure 3A représente une pièce thermoformable de l'orthèse de cheville de la figure 3, selon un mode de réalisation,
la figure 4 représente schématiquement la face latérale interne du pied et de la cheville, équipés de l'orthèse montrée sur la figure 3,
la figure 4A représente une autre pièce thermoformable de l'orthèse de cheville de la figure 3, selon un mode de réalisation,
la figure 5 représente schématiquement une orthèse de pouce selon un mode de réalisation,
la figure 5A représente une pièce thermoformable de l'orthèse de pouce de la figure 5, selon un mode de réalisation,
la figure 6 représente une main et un poignet équipés de l'orthèse de pouce de la figure 5,
la figure 7 représente schématiquement une orthèse correctrice d'hallux valgus selon un mode de réalisation,
la figure 7A représente une pièce thermoformable de l'orthèse correctrice de la figure 7, selon un mode de réalisation,
la figure 8 représente un pied équipé de l'orthèse correctrice de la figure 7.

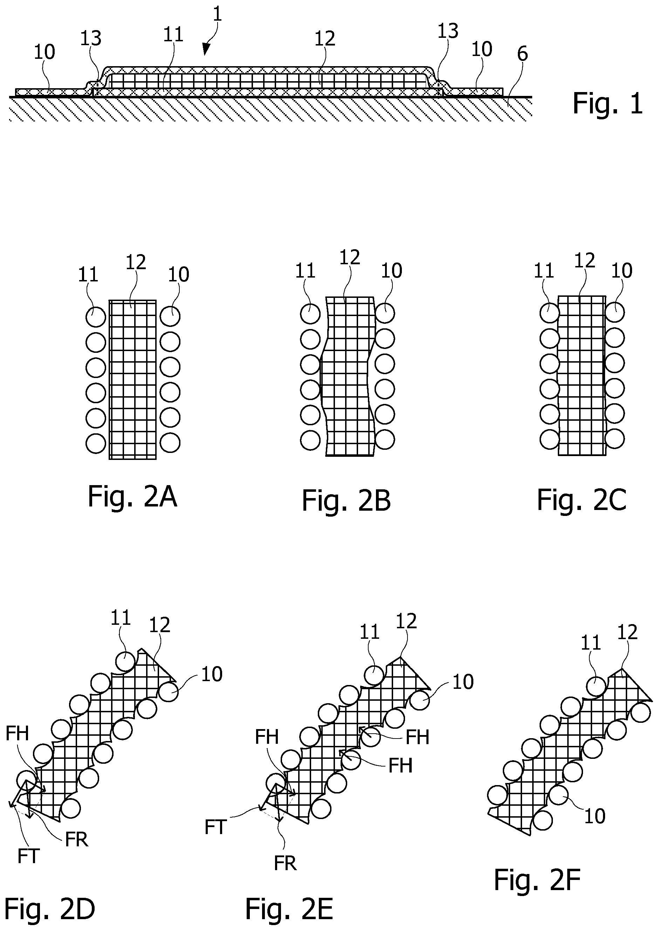

La figure 1 représente une orthèse 1 selon un mode de réalisation. L'orthèse 1 comprend deux couches 10, 11. La couche 10 est au moins en partie élastique, et conformée de manière à former un manchon ajusté autour d'un membre ou d'une articulation à maintenir en y exerçant une certaine pression. La couche 11 est fixée sur la couche 10 de manière à former une poche dans laquelle est insérée une plaquette 12 en un matériau thermoformable. La forme de la poche est ajustée à celle de la plaquette 12. Ainsi le taux de remplissage de la poche par la plaquette peut être compris entre 80 et 95%. La forme de la plaquette 12 peut être elle-même définie en fonction d'une zone du membre ou de l'articulation où le maintien est à assurer.

La couche 10 peut être mise en contact direct avec la peau 6 du patient. La couche 11 peut être fixée sur la couche 10 par une ligne de soudure ou de couture 13, sur l'une ou l'autre des faces de la couche 10.

L'orthèse 1 peut être utilisée de la manière suivante, illustrée par les figures 2A à 2F. Les figures 2A à 2F représentent une partie de la plaquette 12 et des fils formant les couches 10, 11. Avant une première utilisation, l'orthèse (figure 2A) doit subir une opération de thermoformage. A cet effet, elle est chauffée, par exemple en l'immergeant dans de l'eau chaude, avec la plaquette 12 dans la poche, à une température suffisante pour ramollir la plaquette 12. A partir d'un certain moment après son contact avec l'eau chaude, la plaquette 12 devient molle (figure 2B). L'orthèse est alors retirée de l'eau chaude et épongée, ce qui fait pénétrer légèrement les fils des couches 10, 11 dans la plaquette 12 en raison de la ductilité cette dernière à l'état mou (figure 2C). Avant que la plaquette retrouve sa rigidité, le patient enfile l'orthèse autour du membre ou de l'articulation à maintenir en élargissant le manchon formé par la couche 10 (figure 2D)), de préférence en tirant sur les zones sans poche et donc sans plaquette. En raison de sa ductilité, la plaquette 12 se trouve alors légèrement étirée. Les empreintes formées par les fils du tissu dans la plaquette 12 peuvent alors être légèrement élargies. Le manchon est ensuite relâché (figure 2E). La tension élastique exercée par la couche 10 autour du membre ou de l'articulation plaque la plaquette 12 contre la peau du patient. En raison de sa malléabilité, la plaquette 12 prend alors la forme de la zone où elle est plaquée, puis durcit au bout de quelques minutes. Pendant le durcissement de la plaquette (figure 2F), le membre à maintenir est tenu dans la position finale souhaitée. L'orthèse est ainsi adaptée à la morphologie de la zone où elle est appliquée, par le thermoformage de la plaquette 12, réalisé simplement en enfilant l'orthèse sur le membre, par les forces élastiques exercées par la couche 10 assurant le maintien et la déformation de la plaquette 12 préalablement ramollie. Cette opération ne nécessite donc pas l'intervention d'une autre personne et en particulier d'un professionnel. Ce résultat est atteint grâce à la combinaison des propriétés et de la tension du tissu autour du membre, ainsi qu'aux propriétés de l'interface entre le tissu et la plaquette 12, et en particulier au coefficient de frottement entre la plaquette et le tissu et à la ductilité de la plaquette.

Avant l'opération de thermoformage, la partie du membre ou de l'articulation à maintenir peut être recouverte d'un film tel qu'un film plastique pour faciliter le retrait de l'orthèse mouillée à l'issue de l'opération de thermoformage.

La plaquette 12 est par exemple réalisée en un matériau tel que "Aquaplast" fabriqué par la société Patterson, et présente une épaisseur comprise entre 1,5 et 5 mm, par exemple environ 1,6 mm. Ce matériau devient mou à une température comprise entre 65 et 75°C, et reste malléable pendant environ quatre minutes. Ainsi, l'opération de thermoformage de la plaquette 12 peut être répétée autant de fois que nécessaire.

La couche 11 située entre la peau du patient et la plaquette 12 peut être réalisée en un tissu rembourré suffisamment épais (entre 1 et 2 mm d'épaisseur) pour limiter l'inconfort pouvant résulter de l'application d'une plaquette rigide sur la peau. La couche 10 peut être réalisée en un tissu élastique ayant une épaisseur comprise entre 0,2 et 1,5 mm, par exemple environ 0,5 mm. Ces dispositions permettent de réaliser des orthèses légères et peu encombrantes tout en offrant les même services que des orthèses existantes, plus lourdes et encombrantes, et ce sans nécessiter l'intervention d'un professionnel.

Il peut être souhaitable de pouvoir retirer la plaquette de sa poche après thermoformage. Il peut être également souhaitable d'éviter une forte adhérence entre la plaquette 12 et les parties des couches 10, 11 en contact avec la plaquette. En effet, une telle adhérence rend le tissu dans ces parties totalement non élastique et empêche une répartition uniforme de la tension exercée par le manchon formé par la couche 10 autour du membre ou de l'articulation. Une telle adhérence peut également empêcher par la suite un positionnement précis de la plaquette 12 sur le membre ou l'articulation.

Il s'avère que les fils du tissu formant les couches 10 et 11 peuvent s'encastrer dans le matériau de la plaquette 12 en raison de la ductilité de ce dernier, à tel point qu'il peut être très difficile de séparer la plaquette 12 des couches 10, 11 à l'issue de l'opération de thermoformage. Cet effet d'encastrement se produit en particulier lorsque le manchon formé par la couche 10 est élargi pour enfiler l'orthèse autour du membre ou de l'articulation, et durant le durcissement de la plaquette pendant l'opération de thermoformage.

Or les matériaux thermoformables sont généralement très ductiles et cette propriété est recherchée pour épouser une forme aussi précisément que possible. Par conséquent, l'utilisation d'une interface peu ductile entre la plaquette 12 et le tissu 10, 11 affecterait nécessairement la précision du thermoformage, en particulier dans des zones du membre ou de l'articulation présentant de fortes variations de rayon de courbure. Par ailleurs, pour des raisons de confort et de santé, il n'est pas possible de compenser cet effet en augmentant la tension élastique exercée par la couche 10 sur la région du corps recouverte par l'orthèse. Par ailleurs, il existe des normes imposant des valeurs maximum de compression.

La plaquette 12 peut être recouverte d'un film ou d'un revêtement avant de l'insérer dans la poche formée dans l'orthèse. Un traitement de surface tel qu'un laquage peut être également appliqué aux couches 10, 11 à l'intérieur de la poche pour amoindrir le relief formé sur la plaquette par les fils du tissu constituant ces couches, et ainsi réduire l'adhérence du matériau thermoformable que l'on peut constater après l'opération de thermoformage. Le fait d'étirer le manchon, puis de le relâcher durant le thermoformage, lorsque le matériau de la plaquette est ramolli, tend également à élargir les empreintes formées par les fils du tissu dans la plaquette 12.

Selon un mode de réalisation, la plaquette 12 est découpée dans une plaque ayant subi un traitement de surface pour réduire son adhérence avec le tissu des couches 10, 11. La plaquette peut ainsi par exemple être recouverte d'une fine couche en PTFE (Poly Tétra Fluoro Ethylène) . Il en résulte pour les raisons précédemment exposées que la tranche de la plaquette 12 peut adhérer fortement au tissu par encastrement des fils du tissu dans le matériau thermoformable formant la plaquette 12, le coeur du matériau thermoformable ne pouvant être traité anti-adhérent. Si toute la périphérie de la plaquette 12 adhère fortement au tissu, l'élasticité du tissu en contact avec la plaquette 12 ne peut pas être sollicitée, ce qui empêche également une répartition uniforme de la tension élastique exercée par la couche 10 sur la région du corps recouverte par l'orthèse.

Selon un mode de réalisation, une partie de la tranche de la plaquette 12 est recouverte par une bande non adhérente au tissu, formée dans un matériau empêchant les fils des tissus 10, 11 formant la poche de s'encastrer dans la matériau formant la plaquette ou limitant cette incrustation pendant l'opération de thermoformage. Ce matériau peut être par exemple un matériau à base de fibres non tissées. La partie de la tranche de la plaquette 12 recouverte de la bande peut être limitée à un côté de la plaquette en contact avec une zone des couches 10, 11 destinée à être sollicitée en étirement perpendiculaire au côté de la plaquette considéré.

Ainsi, lorsque le manchon est étiré pour enfiler l'orthèse (figure 2D), les fils des couches 10, 11 forment des empreintes dans la plaquette 12. Ces empreintes sont plus larges que le diamètre des fils, car ces derniers exercent à la fois des forces perpendiculaires FH à la surface de la plaquette 12 et des forces tangentielles FT à cette surface. Ainsi, les empreintes n'empêchent pas les mouvements relatifs de la plaquette 12 par rapport au tissu (10, 11) formant la poche. Pendant l'opération de thermoformage proprement dite, le manchon est relâché (figure 2E). Du fait que les empreintes sont plus larges que le diamètre des fils, le tissu (10, 11) formant la poche peut prendre sa place sur la plaquette 12 durant le relâchement du manchon. Ainsi, dans les régions à faible rayon de courbure, les fils des tissus 10, 11 formant la poche exercent une force essentiellement perpendiculaire FH à la surface de la plaquette 12. En s'éloignant d'une telle région, la force exercée par les fils présente une composante tangentielle FT à la surface de la plaquette, qui augmente. La force résultante FR des forces FH et FT plaquent la plaquette 12 sur le membre indépendamment des courbures de la région du membre couverte par la plaquette 12.

Si l'interface entre la plaquette 12 et le tissu n'était pas traitée de manière à éviter l'adhérence, les fils du tissu (10, 11) s'encastreraient dans la plaquette 12 et donc leurs positions respectives seraient figées durant l'étirement et le relâchement du manchon. L'élasticité du tissu dans les régions de contact avec la plaquette 12 serait alors celle de la plaquette qui est très peu élastique à l'état dur.

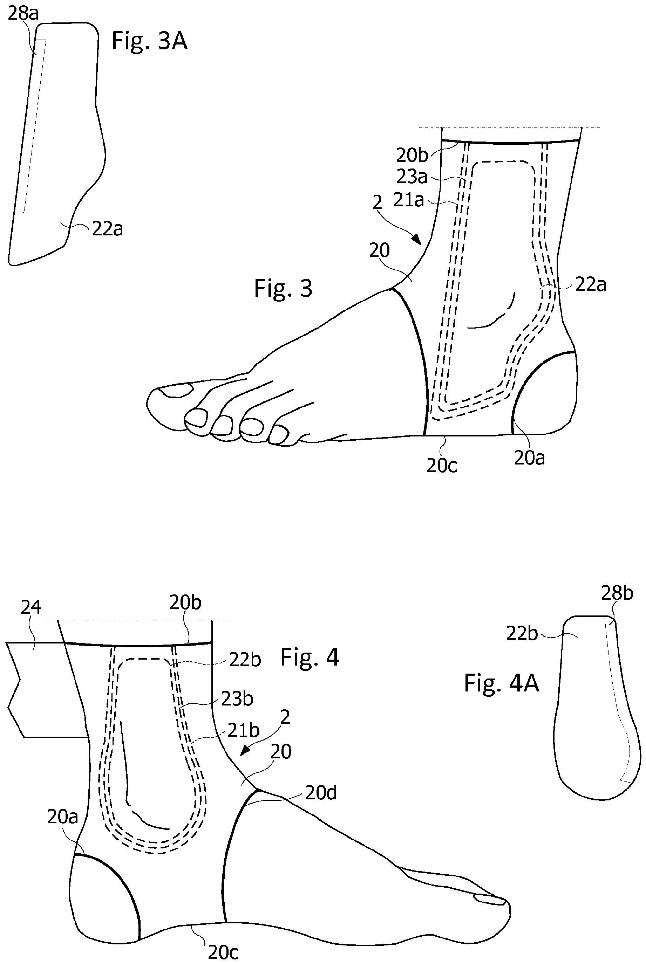

Grâce à ces dispositions, un thermoformage précis de la plaquette 12 peut être obtenu en conformant le manchon correspondant à la couche 10 de manière à ce qu'il exerce une pression sur le membre ou l'articulation comprise entre 2.5 et 5 hPa. Ces valeurs ont été mesurées sur une orthèse de cheville telle que représentée sur les figures 3 et 4.

Les figures 3 et 4 représentent une orthèse 2 adaptée au maintien de la cheville, par exemple à la suite d'un traumatisme. L'orthèse 2 présente la structure de l'orthèse 1 (figure 1). Ainsi, l'orthèse 2 comprend une couche 20 formant un manchon conformé pour serrer la cheville et le pied, avec une ouverture proximale 20b pour le passage de la jambe, une ouverture distale 20d pour le passage du pied et une ouverture intermédiaire 20a pour le passage du talon. La couche 20 est au moins en partie élastique. Une couche 21a est fixée sur la couche 20 de manière à former une première poche dans laquelle est insérée une première plaquette 22a en un matériau thermoformable. La forme de la première poche est ajustée à celle de la plaquette 22a. La première poche est formée sur la couche 20 en un emplacement correspondant à la malléole externe et s'étendant latéralement entre les ouvertures 20a et 20d et entre l'ouverture proximale 20b de la couche 20 et une partie 20c de la couche 20 couvrant un bord latéral du pied entre les zones plantaire et dorsale. La forme de la plaquette 22a est représentée sur la figure 3A. La première poche est formée en fixant la couche 21a sur la couche 20, par une ligne de fixation 23a. La première poche présente par exemple une ouverture le long de l'ouverture proximale 20b du manchon 20, pour insérer ou retirer la plaquette 22a de la poche.

Selon un mode de réalisation, l'orthèse 2 comprend une seconde plaquette 22b en un matériau thermoformable disposée dans une seconde poche formée sur la couche 20 en un emplacement destiné à couvrir la malléole interne et s'étendant latéralement sur la couche 20 autour de la malléole interne jusqu'à l'ouverture 20b du manchon 20. La forme de la plaquette 22b est représentée sur la figure 4A. La seconde poche est formée à l'aide d'une couche de tissu 21b fixée sur le manchon 20, par une ligne de fixation 23b. La seconde poche peut également présenter une ouverture le long de l'ouverture proximale 20b du manchon 20 pour insérer ou retirer la plaquette 22b de la poche.

Si nécessaire, une bande 24 (dont une partie est représentée sur la figure 4) prévue pour être enroulée et serrée autour du pied et de la cheville, peut être utilisée pour serrer davantage l'orthèse autour de la cheville et du pied.

Comme exposé précédemment, une partie de la tranche des plaquettes 22a, 22b peut être recouverte d'une bande 28a, 28b non adhérente aux tissus formant les couches 20, 21a, 21b et empêchant une incrustation des fils de ces tissu de s'incruster dans la tranche des plaquettes 22a, 22b. Dans l'exemple des figures 3A, 4A, les bandes 28a, 28b recouvrent la majeure partie de la partie antérieure (vers l'avant du pied) de la tranche des plaquettes 22a, 22b.

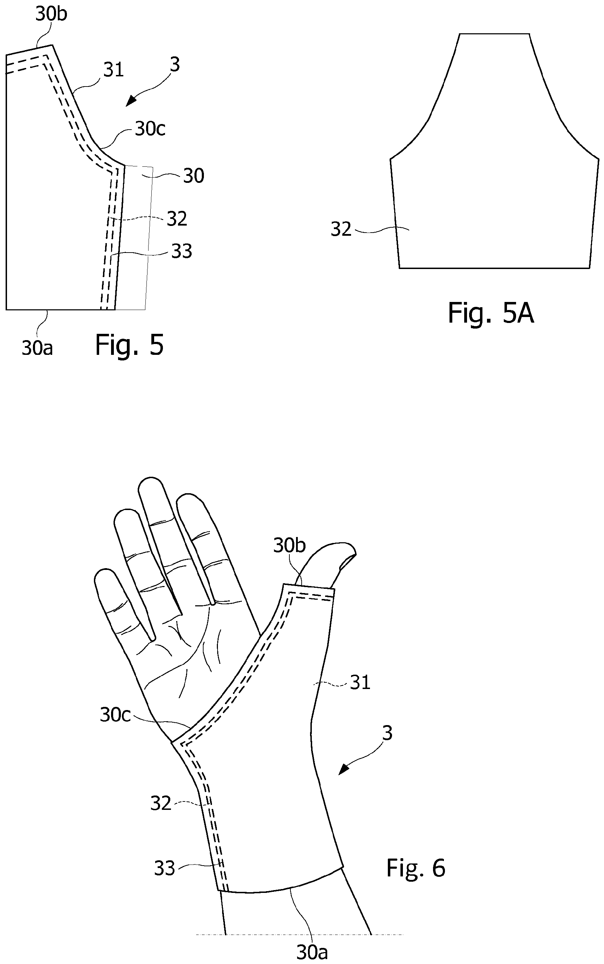

Les figures 5 et 6 représentent une orthèse 3 adaptée au maintien du pouce, notamment pour soulager les patients atteints par la rhizarthrose, en assurant le maintien du pouce en particulier pendant la nuit. L'orthèse 3 présente la structure de l'orthèse 1 (figure 1). Ainsi, l'orthèse 3 comprend deux couches 30, 31. La couche 30 est au moins en partie élastique. La couche 31 est fixée sur la couche 30 de manière à former une poche dans laquelle est insérée une plaquette 32 en un matériau thermoformable. La forme de la poche est ajustée à celle de la plaquette 32. L'orthèse 3 est conformée pour maintenir le pouce et le premier métacarpien en rectitude, selon un mode de réalisation. A cet effet, elle présente la forme d'un manchon conformé pour serrer la base de la main avec le poignet et les deux phalanges du pouce, avec une ouverture proximale 30a pour le passage de l'avant-bras, une ouverture distale 30b pour le passage de l'extrémité distale du pouce et une ouverture intermédiaire 30c pour le passage des autres doigts de la main. La plaquette 32 représentée en particulier sur la figure 5A, est conformée pour recouvrir la première phalange du pouce et l'articulation entre cette première phalange et le premier métacarpien, la base de la main et le poignet, à l'exception de la région du bord ulnaire de la main. La poche est formée sur la couche 30 en un emplacement correspondant aux parties à recouvrir par la plaquette 32. Ainsi, la poche recouvre l'ensemble du manchon à l'exception d'une partie du manchon destinée à couvrir la région du bord ulnaire de la main. La poche est formée en fixant la couche 31 sur la couche 30, par une ligne de fixation 33. La poche présente par exemple une ouverture le long de l'ouverture proximale 30a du manchon 30 pour insérer ou retirer la plaquette 32 de la poche. La partie de la couche 30 non recouverte par la couche 31 est suffisamment élastique pour permettre le retrait de l'orthèse, notamment lorsque la plaquette 32 a été thermoformée. La tension élastique de la couche 30 est suffisante pour plaquer la plaquette 32 contre la peau durant son thermoformage en position sur la main. Une bande peut être prévue pour être associée au manchon de manière à renforcer si nécessaire la compression de thermoformage. Selon un mode de réalisation, cette bande est amovible et enlevée après thermoformage.

Au lieu d'une seule poche et d'une seule plaquette, il peut être prévu de former une poche palmaire et une poche dorsale prévues pour recevoir deux plaquettes en un matériau thermoformable.

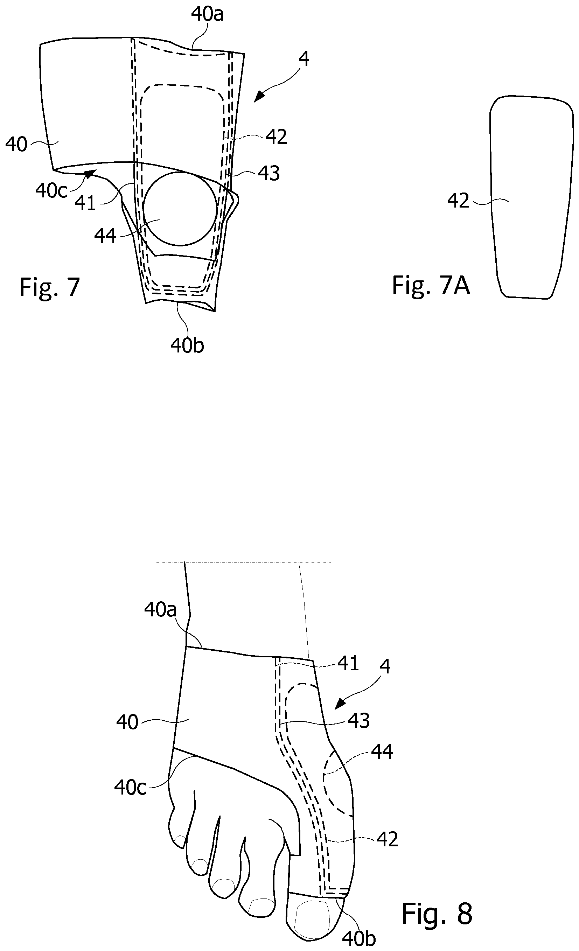

Les figures 7 et 8 représentent une orthèse 4 prévue pour traiter un hallux valgus, notamment pendant la nuit, en exerçant des forces tendant à réaligner la phalange proximale du gros orteil avec le premier métatarsien. A cet effet, l'orthèse 4 est conformée pour maintenir le gros orteil dans le prolongement du premier métatarsien, selon un mode de réalisation. L'orthèse 4 présente la structure de l'orthèse 1 (figure 1). Ainsi, l'orthèse 4 comprend deux couches 40, 41. La couche 40 est au moins en partie élastique. La couche 41 est fixée sur la couche 40 de manière à former une poche dans laquelle est insérée une plaquette 42 en un matériau thermoformable. La forme de la poche est ajustée à celle de la plaquette 42. La couche 40 présente la forme d'un manchon conformé pour serrer le pied, avec une partie entourant le gros orteil, une ouverture proximale 40a pour le passage du pied, une ouverture distale 40b pour le passage de l'extrémité distale du gros orteil et une ouverture intermédiaire 40c pour le passage des autres orteils du pied. La plaquette 42 représentée en particulier sur la figure 7A, est conformée pour recouvrir le bord interne du pied le long du premier métatarsien, et de l'articulation entre le gros orteil et le premier métatarsien, jusqu'à l'articulation entre les phalanges du gros orteil. La poche est formée sur la couche 40 en un emplacement correspondant aux parties à recouvrir par la plaquette 42. La poche est formée en fixant la couche 41 sur la couche 40, par une ligne de fixation 43. La poche présente par exemple une ouverture le long de l'ouverture proximale 40a du manchon 40, pour insérer ou retirer la plaquette 42 de la poche. La partie du manchon 40 non recouverte par la couche 41 est suffisamment élastique pour permettre le retrait de l'orthèse, notamment lorsque la plaquette 42 a été thermoformée. La tension de la couche 40 autour du pied est suffisante pour plaquer la plaquette 42 contre la peau durant l'opération de thermoformage de l'orthèse en position sur le pied. Durant le thermoformage de la plaquette 42, le gros orteil est maintenu autant que possible dans l'axe du premier métatarsien. Une fois thermoformée, la plaquette 42 joue le rôle d'une poutre entre le premier métatarsien et le gros orteil pour exercer une action de réalignement du premier métatarsien et de la phalange proximale du gros orteil. Pour jouer pleinement son rôle de poutre en ayant une grande rigidité, la plaquette peut présenter une épaisseur comprise entre 2 et 5 mm, par exemple environ 3,2 mm. Cependant, il peut être observé sur la figure 8 que la plaquette 42, une fois thermoformée, présente une section courbe dans un plan perpendiculaire à l'axe du pied, c'est-à-dire dans la direction où il est souhaitable que la plaquette présente une grande rigidité. Or cette section courbe contribue à la rigidité de la plaquette 42 suivant cet axe.

Selon un mode de réalisation, la couche 41 formant la poche avec la couche 40 est réalisée en un tissu élastique enduit d'un côté d'une couche en un gel polymère tel qu'un gel de silicone (ou PDMS - polydiméthylsiloxane), présentant une épaisseur comprise entre 0,4 et 0,6 mm. La face du tissu enduite de gel polymère est prévue pour venir en contact avec la peau. Le gel polymère employé présentant un coefficient de frottement avec la peau plus élevé que celui du tissu composant la couche 41, cette couche de gel polymère permet d'éviter que l'orthèse glisse sur la peau vers l'avant ou vers l'arrière du pied, ou autour du gros orteil, et donc que la plaquette 42 soit mal positionnée. Il est préférable que le tissu enduit de gel polymère présente une élasticité dans le sens perpendiculaire à l'axe du pied supérieure ou égale à l'élasticité du tissu dans le sens parallèle à l'axe du pied.

Selon un mode de réalisation, une pastille 44 souple est disposée avant le thermoformage, sur la couche 41 (à l'intérieur de l'orthèse), sur une zone destinée à couvrir l'articulation entre le premier métatarsien et le gros orteil. La pastille 44 est retirée après le thermoformage de manière à laisser un vide permettant d'éviter que l'orthèse exerce une pression sur l'articulation entre le gros orteil et le premier métatarsien. Une nouvelle opération de thermoformage de la plaquette 42 en présence de la pastille 44 peut être effectuée si nécessaire, pour ajuster les forces de réalignement exercées par l'orthèse, et poursuivre l'action correctrice de l'orthèse.

La pastille 44 peut présenter une forme lenticulaire et être réalisée en un gel polymère lisse, telle que le gel de silicone lisse. Ainsi, en raison des propriétés du gel de silicone, la pastille 44 adhère naturellement à la couche de gel polymère également lisse, déposée sur la couche 41. Cet effet d'adhésion permet de garantir le maintien en place de la pastille 44 en particulier lorsque l'orthèse est immergée dans l'eau chaude pour procéder à l'opération de thermoformage. Cet effet d'adhésion peut être renforcé en prévoyant que la pastille 44 présente une face concave de manière à obtenir un effet de ventouse. Une telle face concave peut être aisément obtenue si la pastille est formée à partir d'un liquide versé dans un moule, puis polymérisé. En effet, la tension superficielle du liquide fait remonter la surface du liquide le long du bord du moule.

Les orthèses 2 à 4 qui viennent d'être décrites sont ajustées à la zone du membre ou de l'articulation à maintenir. La forme de la plaquette thermoformable est également ajustée à la zone à maintenir. Par ailleurs, la plaquette est disposée dans une poche fixe par rapport à l'orthèse et dont les dimensions correspondent à celles de la plaquette, de sorte que la plaquette ne peut pas bouger dans la poche. Ces dispositions permettent d'assurer que la plaquette soit positionnée correctement par rapport au membre ou à l'articulation à maintenir. Il en résulte que l'adaptation de l'orthèse à un patient ne nécessite pas l'intervention d'un professionnel. En outre, le thermoformage de la plaquette peut être effectué simplement en enfilant l'orthèse avec la plaquette chauffée directement sur le membre ou l'articulation à maintenir, la position de l'orthèse sur le membre ou l'articulation à maintenir étant définie par la forme de l'orthèse. Il en résulte que l'opération de thermoformage peut être effectuée directement par le patient lui-même sans l'aide d'une autre personne. Pour tenir compte des variations de morphologie d'une personne à une autre, il peut être prévu de fabriquer chaque type d'orthèse en plusieurs tailles, par exemple 3 ou 4 tailles différentes.

Il apparaîtra clairement à l'homme de l'art que la présente invention est susceptible de diverses variantes de réalisation et diverses applications. En particulier, l'invention n'est pas limitée aux applications décrites, mais peut s'appliquer à tout autre membre ou articulation, y compris un membre ou une articulation d'un animal vertébré.

Par ailleurs, d'autres matériaux que des tissus peuvent être utilisés pour réaliser le manchon et la poche, tels que des films ou membranes microporeux assemblés par des soudures.

## Revendications

1. Orthèse pour le maintien d'un membre ou d'une articulation d'un humain ou d'un animal vertébré, comprenant :
un manchon (10) formé dans un tissu élastique, et conformé pour serrer un membre ou une articulation,
une plaquette (12) en un matériau thermoformable, et
une poche formée sur le manchon pour recevoir la plaquette, la poche présentant une forme ajustée à celle de la plaquette,
**caractérisée en ce que** la surface de la plaquette (12) et/ou la surface intérieure de la poche sont traitées pour amoindrir un effet d'encastrement de fils du tissu (10, 11) formant la poche, dans la plaquette, et ainsi autoriser un mouvement relatif de la plaquette par rapport au tissu formant la poche, pendant et après une opération de thermoformage de la plaquette, réalisée en plaçant l'orthèse sur le membre ou l'articulation avec la plaquette présente dans la poche.

2. Orthèse selon la revendication 1, dans laquelle la poche est formée par une couche (11) fixée sur une face intérieure du manchon (10), destinée à venir en contact avec la peau du membre ou l'articulation à maintenir.

3. Orthèse selon la revendication 1 ou 2, dans laquelle la poche est formée par une couche (11) fixée sur le manchon (10), réalisée en un tissu rembourré.

4. Orthèse selon l'une des revendications 1 à 3, dans laquelle la partie du manchon (10) non recouverte par la poche est réalisée dans un tissu élastique.

5. Orthèse selon l'une des revendications 1 à 4, dans laquelle le manchon (10) est réalisé dans un tissu élastique présentant une épaisseur comprise entre 0,2 et 1,5 mm.

6. Orthèse selon l'une des revendications 1 à 5, dans laquelle la plaquette (12) et/ou les faces intérieures de la poche destinées à venir en contact avec la plaquette sont recouvertes d'un film ou d'une enduction pour réduire l'adhérence entre la plaquette et les faces intérieures de la poche pendant et à la suite d'une opération de thermoformage de la plaquette durant laquelle la plaquette est placée dans la poche.

7. Orthèse selon l'une des revendications 1 à 5, dans laquelle la plaquette (12) est découpée dans une feuille ayant subi un traitement antiadhérent, et une partie de la tranche de la plaquette est recouverte d'une bande non adhérente aux faces intérieures de la poche et limitant l'adhérence entre la plaquette et les faces intérieures de la poche pendant et à la suite d'une opération de thermoformage de la plaquette.

8. Orthèse selon l'une des revendications 1 à 7, dans laquelle le manchon (20) est adapté à l'une des formes suivantes :
la forme de la cheville et du pied d'un humain, avec une ouverture proximale (20b) pour le passage de la jambe, une ouverture distale (20d) pour le passage de l'avant du pied et une ouverture intermédiaire (20a) pour le passage du talon, le manchon comprenant deux poches (21a, 21b) destinées à couvrir les malléoles de la cheville en s'étendant entre la base du pied (20c) et l'ouverture proximale du manchon, les deux poches étant prévues pour recevoir deux plaquettes (22a, 22b) en un matériau thermoformable ;
la forme du poignet d'un humain et d'une partie proximale de la main et du pouce, avec une ouverture distale (30b) pour le passage de la partie distale du pouce, une ouverture proximale (30a) pour le passage de l'avant-bras et une ouverture intermédiaire (30c) pour le passage des doigts de la main, le manchon comprenant une poche (31) destinée à couvrir l'ensemble du manchon à l'exception d'une partie du manchon destinée à couvrir le tranchant de la main, la poche étant prévue pour recevoir la plaquette (32) en un matériau thermoformable ;
la forme du pied et d'une partie proximale du gros orteil d'un humain, avec une ouverture distale (40b) pour le passage de la partie distale du gros orteil, une ouverture proximale (40a) pour le passage du pied et une ouverture intermédiaire (40c) pour le passage des autres orteils du pied, le manchon comprenant une poche (41) sur une partie du manchon destinée à couvrir une partie de la face latérale interne du pied et gros orteil, la poche étant prévue pour recevoir la plaquette (42) en un matériau thermoformable.

9. Orthèse selon la revendication 8, dans laquelle le manchon (20) est adapté à la forme de la cheville et du pied d'un humain, le manchon étant solidaire d'une bande (24) prévue pour être enroulée autour du pied et de la cheville, pour serrer davantage le manchon autour du pied et de la cheville, notamment durant le thermoformage de la plaquette.

10. Orthèse selon la revendication 8, dans laquelle une face externe de la poche (41), destinée à venir en contact avec la peau du membre ou l'articulation à maintenir, est recouverte d'une enduction en gel polymère, la partie de la poche enduite de gel polymère présentant une élasticité dans le sens perpendiculaire à l'axe du membre, supérieure ou égale celle dans un sens parallèle à l'axe du membre.

11. Orthèse selon la revendication 10, comprenant une pastille (44) en gel polymère fixée de manière amovible contre la face externe de la poche (41) recouverte d'une enduction en gel polymère.

12. Procédé de fabrication d'une orthèse pour le maintien d'un membre ou d'une articulation d'un humain ou d'un animal vertébré, comprenant des étapes consistant à :
fabriquer un manchon (10) en tissu partie élastique, ajusté pour serrer un membre ou une articulation,
ajuster la forme d'une plaquette (12) en un matériau thermoformable à la celle d'une partie du membre ou de l'articulation, et
former une poche sur le manchon pour recevoir la plaquette, la poche présentant une forme ajustée à celle de la plaquette,
**caractérisé en ce qu'**il comprend une étape de traitement de la surface de la plaquette (12) et/ou de la surface intérieure de la poche pour amoindrir un effet d'encastrement de fils du tissu (10, 11) formant la poche, dans la plaquette, et ainsi autoriser un mouvement relatif de la plaquette par rapport au tissu formant la poche, pendant et après une opération de thermoformage de la plaquette, réalisée en plaçant l'orthèse sur le membre ou l'articulation avec la plaquette présente dans la poche.

13. Procédé selon la revendication 12, dans lequel la formation de la poche est réalisée en fixant une couche (11) sur une face intérieure du manchon (10), destinée à venir en contact avec la peau du membre ou l'articulation à maintenir.

14. Procédé selon l'une des revendications 12 et 13, et de disposition de la plaquette (12) dans la poche, ainsi qu'une opération de thermoformage de l'orthèse comprenant des étapes consistant à : chauffer l'orthèse à une température suffisante pour ramollir la plaquette, et placer le manchon autour du membre ou de l'articulation avant que la plaquette durcisse.

15. Procédé selon la revendication 14, dans lequel l'opération de thermoformage comprend une étape de couverture du membre ou de l'articulation par un film avant de placer le manchon autour du membre ou de l'articulation.

16. Procédé selon la revendication 14 ou 15, dans lequel l'opération de thermoformage comprend une étape de placement d'une pastille (44) sur une zone de la face extérieure de la poche destinée à venir en contact avec la peau, avant de placer le manchon autour du membre ou de l'articulation, la pastille étant retirée du manchon après l'opération de thermoformage, de manière à ménager un espace vide entre la plaquette et la peau.

## Patentansprüche

1. Orthese zum Halten eines Gliedes oder Gelenks eines Menschen oder eines Wirbeltieres, umfassend:
eine Hülse (10), die in einem elastischen Gewebe ausgebildet und geformt ist, um ein Glied oder ein Gelenk zu schienen,
eine Platte (12) aus einem thermoformbaren Material und
eine auf der Hülse gebildete Tasche zur Aufnahme der Platte, wobei die Tasche eine an die Form der Platte angepasste Form aufweist,
**dadurch gekennzeichnet, dass** die Oberfläche der Platte (12) und/oder die Innenfläche der Tasche behandelt sind, um einen Einbettungseffekt von Fäden des die Tasche bildenden Gewebes (10, 11) in der Platte zu reduzieren und somit eine Relativbewegung der Platte in Bezug auf das die Tasche bildende Gewebe während und nach einem Thermoformvorgang der Platte zu ermöglichen, der durch Platzieren der Orthese auf dem Glied oder dem Gelenk mit der in der Tasche vorhandenen Platte durchgeführt wird.

2. Orthese nach Anspruch 1, wobei die Tasche durch eine Schicht (11) gebildet ist, die an einer Innenfläche der Hülse (10) befestigt ist und dazu bestimmt ist, mit der Haut des zu haltenden Gliedes oder Gelenks in Kontakt zu kommen.

3. Orthese nach Anspruch 1 oder 2, wobei die Tasche durch eine Schicht (11) gebildet ist, die an der Hülse (10) befestigt ist und aus einem gepolsterten Gewebe hergestellt ist.

4. Orthese nach einem der Ansprüche 1 bis 3, wobei der nicht durch die Tasche abgedeckte Teil der Hülse (10) aus einem elastischen Gewebe besteht.

5. Orthese nach einem der Ansprüche 1 bis 4, wobei die Hülse (10) aus einem elastischen Gewebe aufweisend eine Dicke zwischen 0,2 und 1,5 mm hergestellt ist.

6. Orthese nach einem der Ansprüche 1 bis 5, wobei die Platte (12) und/oder die Innenflächen der Tasche, die dazu bestimmt sind, mit der Platte in Kontakt zu kommen, mit einer Folie oder Beschichtung bedeckt sind, um die Haftung zwischen der Platte und den Innenflächen der Tasche während und nach einem Thermoformvorgang der Platte, bei dem die Platte in die Tasche gelegt wird, zu verringern.

7. Orthese nach einem der Ansprüche 1 bis 5, wobei die Platte (12) aus einem Blatt geschnitten wird, das einer Antihaftbehandlung unterzogen wurde, und ein Abschnitt der Plattenkante mit einem nicht haftenden Streifen an den Innenseiten der Tasche bedeckt ist und die Haftung zwischen der Platte und den Innenseiten der Tasche während und nach einem Thermoformvorgang der Platte begrenzt.

8. Orthese nach einem der Ansprüche 1 bis 7, wobei die Hülse (20) an eine der folgenden Formen angepasst ist:
die Form eines menschlichen Knöchels und Fußes mit einer proximalen Öffnung (20b) für den Durchgang des Beines, einer distalen Öffnung (20d) für den Durchgang des Vorderfußes und einer Zwischenöffnung (20a) für den Durchgang der Ferse, wobei die Hülse zwei Taschen (21a, 21b) zum Abdecken der sich zwischen der Basis des Fußes (20c) und der proximalen Öffnung der Hülse erstreckenden Knöchelmaleolen umfasst, wobei beide Taschen zur Aufnahme von zwei Platten (22a, 22b) aus einem thermoformbaren Material vorgesehen sind;
die Form des Handgelenks eines Menschen und eines proximalen Teils der Hand und des Daumens, aufweisend eine distale Öffnung (30b) für den Durchgang des distalen Teils des Daumens, eine proximale Öffnung (30a) für den Durchgang des Unterarms und eine Zwischenöffnung (30c) für den Durchgang der Finger der Hand, wobei die Hülse eine Tasche (31) zum Abdecken der gesamten Hülse mit Ausnahme eines Teils der Hülse zum Abdecken des Handrandes umfasst, wobei die Tasche zur Aufnahme der Platte (32) aus einem thermoformbaren Material vorgesehen ist;
die Form des Fußes und eines proximalen Abschnitts der Großzehe eines Menschen, mit einer distalen Öffnung (40b) für den Durchgang des distalen Abschnitts der Großzehe, einer proximalen Öffnung (40a) für den Durchgang des Fußes und einer Zwischenöffnung (40c) für den Durchgang der anderen Zehen des Fußes, wobei die Hülse eine Tasche (41) an einem Abschnitt der Hülse zum Abdecken eines Abschnitts der inneren Seitenfläche des Fußes und der Großzehe umfasst, wobei die Tasche zur Aufnahme der Platte (42) aus einem thermoformbaren Material vorgesehen ist.

9. Orthese nach Anspruch 8, wobei die Hülse (20) an die Form eines menschlichen Knöchels und Fußes angepasst ist, wobei die Hülse einstückig mit einem Streifen (24) ausgebildet ist, der dazu bestimmt ist, um den Fuß und den Knöchel gewickelt zu werden, um die Hülse um den Fuß und den Knöchel herum weiter zu schienen, insbesondere beim Thermoformen der Platte.

10. Orthese nach Anspruch 8, wobei eine Außenfläche der Tasche (41), die dazu bestimmt ist, mit der Haut des zu haltenden Gliedes oder Gelenks in Kontakt zu kommen, mit einer Polymergel-Beschichtung bedeckt ist, wobei der mit Polymergel beschichtete Teil der Tasche eine Elastizität in der Richtung senkrecht zur Achse des Gliedes aufweist, die größer oder gleich derjenigen in einer Richtung parallel zur Achse des Gliedes ist.

11. Orthese nach Anspruch 10, umfassend ein Polymergel-Plättchen (44), das abnehmbar an der Außenfläche der Tasche (41) befestigt ist, die mit einer Polymergel-Beschichtung bedeckt ist.

12. Verfahren zur Herstellung einer Orthese zum Halten eines Gliedes oder Gelenks eines Menschen oder Wirbeltieres, umfassend die folgenden Schritte:
Herstellen einer Hülse (10) aus elastischem Teilgewebe, die so eingestellt ist, dass sie ein Glied oder Gelenk schient,
Anpassen der Form einer Platte (12) aus einem thermoformbaren Material an die eines Teils des Gliedes oder des Gelenks, und
Bilden einer Tasche auf der Hülse zur Aufnahme der Platte, wobei die Tasche eine an die Platte angepasste Form aufweist,
**dadurch gekennzeichnet, dass** es einen Schritt zum Behandeln der Oberfläche der Platte (12) und/oder der Innenfläche der Tasche umfasst, um einen Einbettungseffekt von Fäden des die Tasche bildenden Gewebes (10, 11) in der Platte zu reduzieren und so eine Relativbewegung der Platte in Bezug auf das die Tasche bildende Gewebe während und nach einem Thermoformvorgang der Platte zu ermöglichen, der durch Platzieren der Orthese auf dem Glied oder dem Gelenk mit der in der Tasche vorhandenen Platte durchgeführt wird.

13. Verfahren nach Anspruch 12, bei dem die Bildung der Tasche durch Fixieren einer Schicht (11) auf einer Innenseite der Hülse (10) durchgeführt wird, die dazu bestimmt ist, mit der Haut des zu haltenden Gliedes oder Gelenks in Kontakt zu kommen.

14. Verfahren nach einem der Ansprüche 12 und 13 und zum Anordnen der Platte (12) in der Tasche sowie ein Thermoformvorgang der Orthese, umfassend die Schritte: Erwärmen der Orthese auf eine Temperatur, die ausreicht, um die Platte weich zu machen, und Platzieren der Hülse um das Glied oder Gelenk herum, bevor die Platte aushärtet.

15. Verfahren nach Anspruch 14, wobei der Thermoformvorgang einen Schritt umfasst, bei dem das Glied oder das Gelenk mit einer Folie abgedeckt wird, bevor die Hülse um das Glied oder das Gelenk gelegt wird.

16. Verfahren nach Anspruch 14 oder 15, wobei der Thermoformvorgang einen Schritt des Anordnens eines Plättchens (44) auf einem Bereich der Außenfläche der Tasche umfasst, der dazu bestimmt ist, mit der Haut in Kontakt zu kommen, bevor das Plättchen um den Arm oder das Gelenk gelegt wird, wobei das Plättchen nach dem Thermoformvorgang aus der Hülse entfernt wird, um so einen Hohlraum zwischen dem Plättchen und der Haut bereitzustellen.

## Claims

1. An orthosis for maintaining a limb or joint of a human or vertebrate animal, comprising:
• a sleeve (10) formed in an elastic fabric, and shaped to compress a limb or joint,
• a plate (12) made of a thermoformable material, and
• a pocket formed on the sleeve for receiving the plate, the pocket having a shape adjusted to that of the plate,
• **characterized in that** the surface of the plate (12) and/or the inner surface of the pocket are treated to reduce the embedding effect of threads of the fabric (10, 11) forming the pocket, in the plate, and thereby allow relative movement of the plate with respect to the fabric forming the pocket, during and after a thermoforming operation of the plate, performed by placing the orthosis on the limb or articulation with the plate present in the pocket.

2. The orthosis according to claim 1, wherein the pocket is formed by a layer (11) attached on an inner face of the sleeve (10), configured to contact the skin of the limb or the joint to be maintained.

3. The orthosis according to claim 1 or 2, wherein the pocket is formed by a layer (11) attached to the sleeve (10), made of a padded fabric.

4. The orthosis according to one of claims 1 to 3, wherein the portion of the sleeve (10) not covered by the pocket is made of an elastic fabric.

5. The orthosis according to one of claims 1 to 4, wherein the sleeve (10) is made of an elastic fabric having a thickness between 0.2 and 1.5 mm.

6. The orthosis according to one of claims 1 to 5, wherein the plate (12) and/or the inner faces of the pocket configured to contact the plate are covered with a film or a coating to reduce the adhesion between the plate and the inner faces of the pocket during and following a thermoforming operation of the plate while the plate is placed in the pocket.

7. The orthosis according to one of claims 1 to 5, wherein the plate (12) is cut from anti-adhesion-treated sheet material, and a portion of the plate edges is covered with a strip that does not adhere to the inner faces of the pocket and limits the adhesion between the plate and the inner faces of the pocket during and after a thermoforming operation of the plate.

8. The orthosis according to one of claims 1 to 7, wherein the sleeve (20) is adapted to one of the following shapes:
• the shape of the ankle and foot of a human, with a proximal opening (20b) for the passage of the leg, a distal opening (20d) for the passage of the forefoot, and an intermediate opening (20a) for the passage of the heel, the sleeve including two pockets (21a, 21b) for covering the malleoli of the ankle extending between the base of the foot (20c) and the proximal opening of the sleeve, the two pockets being configured to receive two plates (22a, 22b) of a thermoformable material;
• the shape of the wrist and a proximal part of the hand and thumb of a human, with a distal opening (30b) for the passage of the distal portion of the thumb, a proximal opening (30a) for the passage of the forearm, and an intermediate opening (30c) for the passage of the fingers of the hand, the sleeve including a pocket (31) for covering the entire sleeve except for a portion of the sleeve that covers the side of the hand, the pocket being configured to receive the plate (32) of a thermoformable material;
• the shape of the foot and a proximal portion of the big toe of a human, with a distal opening (40b) for the passage of the distal portion of the big toe, a proximal opening (40a) for the passage of the foot, and an intermediate opening (40c) for the passage of the other toes of the foot, the sleeve including a pocket (41) on a portion of the sleeve configured to cover a portion of the internal lateral face of the foot and big toe, the pocket being configured to receive the plate (42) of a thermoformable material.

9. The orthosis according to claim 8, wherein the sleeve (20) is adapted to the shape of the ankle and the foot of a human, the sleeve being secured to a band (24) configured to be wrapped around the foot and the ankle, to further tighten the sleeve around the foot and the ankle, especially during the thermoforming of the plate.

10. The orthosis according to claim 8, wherein an outer face of the pocket (41), configured to contact the skin of the limb or the joint to be maintained, is coated with a polymer gel layer, the portion of the pocket coated with polymer gel having an elasticity in the direction perpendicular to the axis of the limb, greater than or equal to that in a direction parallel to the axis of the limb.

11. The orthosis according to claim 10, comprising a polymer gel pellet (44) removably attached to the outer surface of the pocket (41) coated with a polymer gel.

12. A method of manufacturing an orthosis for maintaining a limb or joint of a human or vertebrate animal, comprising steps of:
• fabricating a partially elastic fabric sleeve (10), shaped to compress a limb or joint,
• adjusting the shape of a plate (12) of a thermoformable material to a portion of the limb or joint, and
• forming a pocket on the sleeve for receiving the plate, the pocket having a shape adjusted to that of the plate,
• **characterized in that** it comprises a step of treating the surface of the plate (12) and/or the inner surface of the pocket to reduce the embedding effect of threads of the fabric (10, 11) forming the pocket, in the plate, and thus allow relative movement of the plate with respect to the fabric forming the pocket, during and after a thermoforming operation of the plate, performed by placing the orthosis on the limb or articulation with the plate present in the pocket.

13. The method of claim 12, wherein the formation of the pocket is performed by attaching a layer (11) on an inner face of the sleeve (10), configured to contact the skin of the limb or the joint to be maintained.

14. A method according to one of claims 12 and 13 and placing the plate (12) in the pocket, as well as a thermoforming operation of the orthosis comprising the steps of: heating the orthosis to a temperature sufficient to soften the plate, and placing the sleeve around the limb or joint before the plate hardens.

15. The method of claim 14 wherein the thermoforming operation comprises a step of covering the limb or joint with a film prior to placing the sleeve around the limb or joint.

16. The method as claimed in claim 14 or 15, wherein the thermoforming operation comprises a step of placing a pellet (44) on an area of the outer face of the pocket configured to contact the skin, before placing the sleeve around the limb or the joint, the pellet being removed from the sleeve after the thermoforming operation, so as to form a gap between the plate and the skin.
